# EUROPEAN PATENT APPLICATION

(11) **EP 1 326 074 A1**
(43) Date of publication of application: **09.07.2003**
(21) Application number: 02075023.8
(22) Date of filing: 07.01.2002
(51) Int. Cl.: G01N 33/03

(54) **Device suitable for analysing edible oil quality**

(71) Applicant: UNILEVER N.V., 3013 AL Rotterdam (NL)
(72) Inventor: Appel, Adrianus C.M., Unilever R&D Vlaardingen, 3133 AT Vlaardingen (NL); Hage, Ronald, Unilever R&D Vlaardingen, 3133 AT Vlaardingen (NL); Lienke, Joachim, Unilever R&D Vlaardingen, 3133 AT Vlaardingen (NL)
(74) Representative: Kleiborn, Paul Erik

(57) **Abstract**

The invention relates to a device suitable for analysing edible oil quality, comprising an indicator composition comprising a colorant and a bleach catalyst. The invention further relates to a method for analysing oil quality, wherein the amount of hydroperoxides in the oil is measured and the amount of hydroperoxides is used to express the oil quality.

## Description

### Field of the invention

The invention relates to a device suitable for analysing edible oil quality. The invention further relates to a method for analysing edible oil quality.

### Background of the invention

Edible oils (herein including oils, fats, shortening and mixtures thereof) may be used to fry food. The frying of food in the oil is usually conducted at high temperatures. In deep frying, the frying oil is used more than once and during its lifetime the quality of the oil gradually deteriorates, mainly due to thermal degradation. The degradation products, produced during thermal degradation may be harmful for the human health and therefore the frying oil is discarded as soon as the level of degradation of the oil becomes to high. On the other hand one wants to use the frying oil as many cycles as possible, because the frying oil forms a cost factor in the preparation of fried food.

A number of methods and devices have been suggested to measure frying oil quality. One prior art method is to measure the specific electric resistance of the frying oil. Such a method is disclosed in EP-A-640834. As the oil deteriorates the electric resistance of the the oil decreases. This method has the disadvantage that the electric resistance of different fresh oils may largely vary, such that a calibration of the measuring device with fresh frying oil is needed.

Another method is known from US-A-5420041, wherein deterioration is determined by analysing the acid value of the oil with an infrared spectrometer. This method involves a complicated procedure, involving an infrared spectrometer, in which an infrared spectrum has to be analysed.

Still another method is known from WO 00/52448, which involves draining time of the oil. The result of this method depends on the viscosity of the oil, and when different oils are used, calibration with fresh oil is needed.

From WO 99/005519 a method and device to measure frying oil quality is known that involves quantifying the total amount of of polar compounds in the oil. This method has the advantage that it measure the amount of polar compound directly and no calibration with fresh oil is needed. A disadvantage is that method and device are difficult to use in practice. The temperature of the device, as shown in figure 4, needs to be carefully controlled. Further a drop of the frying oil has to be deposited on the test strip placed. Both steps may be cumbersome in a home kitchen or in a commercial kitchen, where cooking may take place at the same time.

### Summary of the invention

An object of the invention is to provide a method and device for measuring edible frying oil quality. A further object of the invention is to provide such a method and device that is simple to use in a kitchen or factory. Another object is to provide such a method and device that does not need calibration with fresh oil. Still another object is to provide such a method and device that measures oil quality in a reproducible and/or accurate way. One or more of these objects are attained according to the invention in that it provides a method for analysing oil quality, wherein the amount of hydroperoxides in the oil is measured and the amount of hydroperoxides used to express the oil quality.

Preferably the the amount of hydroperoxides in the oil is measured using a colorant, wherein the time needed to change the color of the colorant is taken as a measure for the amount of hydroperoxides in the oil. The invention further relates to a method for analysing oil quality, wherein the amount of antioxidants in the oil is measured and the amount of antioxidants is used to express the oil quality. Preferably, the amount of antioxidants in the oil is measured using a colorant, wherein the time prior to the start of change of color of the colorant is taken as a measure for the amount of antioxidants in the oil. The invention further provides a device suitable for analysing edible oil quality comprising an indicator composition comprising a colorant and a bleach catalyst.

### Detailed description of the invention

Oil and fat may be used herein interchangebly. Oil herein includes oils, fats, shortening and mixtures thereof.

The device according to the invention comprises an indicator composition comprising a colorant and a bleach catalyst.

### Colorant

The colorant may be any colorant or mixture of colorants, that changes color under the unfluence of the bleach catalyst, in the presence of hydroperoxide. Preferably the change of color may be observed by humans, more preferably by humans with the naked eye. Preferably, the change of color is bleaching.

The colorant may be a natural or synthetic dye. Examples of groups of colorants that may be used are: azo dyestuffs, (having the general formula RN=NR'); congo red; azoic dyes; acetate dyes, anthraquinone dyes. Preferably the colorant is a hydrophobic colorant.

Preferably the the colorant is a food colorant. Food colorants include natural colours, derived primarily from vegetable sources and sometimes called vegetable dyes; inorganic pigments; combinations of organic and metallic compounds (called lakes); and synthetic coal-tar substances.

Preferably, food colors used according to the invention are those that are allowed as food colorant in the country where the invention is used, such as for the United States, the Federal Food, Drug, and Cosmetic Act. Many other countries have similar food colouring controls. Preference is given to natural, or vegetable, colourings, which are generally considered safe.

Most preferably the food colorant is chosen from the group consisting of curcumin, riboflavin, tartrazin, Chinolin, Cochenil, and beta-carotene.

### Bleach catalyst

The bleach catalyst per se may be selected from a wide range of transition metal complexes of organic molecules (ligands). Suitable organic molecules (ligands) for forming complexes and complexes thereof are found, for example in: GB 9906474.3; GB 9907714.1; GB 98309168.7, GB 98309169.5; GB 9027415.0 and GB 9907713.3; DE 19755493; EP 999050; WO-A-9534628; EP-A-458379; EP 0909809; United States Patent 4,728,455; WO-A-98/39098; WO-A-98/39406, WO 9748787, WO 0029537; WO 0052124, and WO0060045 the complexes and organic molecule (ligand) precursors of which are herein incorporated by reference. The bleach catalysts in aforementioned references are discussed with reference to "air bleaching" however the catalysts are equally applicable for bleaching with a peroxy source. With regard to other catalysts and further discussion concerning the use of transition metal complexes as catalysts to activate the peroxy bleaching the reader is directed to United States Patent 4728455, EP-A-0458379, WO-A-9534628, WO-A-9718035, and WO-A-9748787.

The ligand forms a complex with one or more transition metals, in the latter case for example as a dinuclear complex. Suitable transition metals include for example: manganese in oxidation states II-V, iron II-V, copper I-III, cobalt I-III, titanium II-IV, tungsten IV-VI, vanadium II-V and molybdenum II-VI.

A bleaching composition comprising:
a) a monomer ligand or transition metal catalyst thereof of a ligand having the formula (I) : 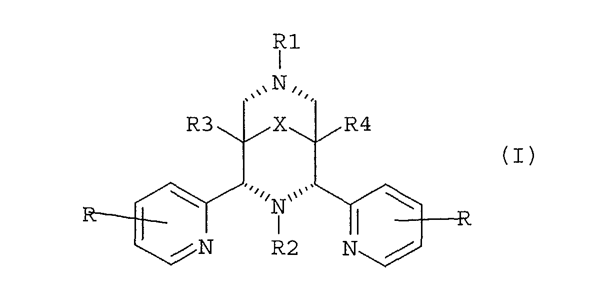
wherein each R is independently selected from: hydrogen, F, Cl, Br, hydroxyl, C1-C4-alkylO-, -NH-CO-H, -NH-CO-C1-C4-alkyl,-NH2, -NH-C1-C4-alkyl, and C1-C4-alkyl;
R1 and R2 are independently selected from:
C1-C4-alkyl,
C6-C10-aryl, and,
a group containing a heteroatom capable of coordinating to a transition metal, wherein at least one of R1 and R2 is the group containing the heteroatom;
R3 and R4 are independently selected from hydrogen, C1-C8 alkyl, C1-C8-alkyl-O-C1-C8-alkyl, C1-C8-alkyl-O-C6-C10-aryl, C6-C10-aryl, C1-C8-hydroxyalkyl, and - (CH2)ₙC(O)OR5
wherein R5 is independently selected from: hydrogen, C1-C4-alkyl, n is from 0 to 4, and mixtures thereof; and,
X is selected from C=O, -[C(R6)₂]_{y}- wherein Y is from 0 to 3 each R6 is independently selected from hydrogen, hydroxyl, C1⁻ C4-alkoxy and C1-C4-alkyl.

The ligand forms a complex with one or more transition metals, in the latter case for example as a mononuclear or dinuclear complex. Suitable transition metals include for example:
manganese in oxidation states II-V, iron II-V, copper I-III, cobalt I-III, titanium II-IV, tungsten IV-VI, vanadium II-V and
molybdenum II-VI. Preferably the bleach catalyst comprises Fe or Co, more preferably it comprises Fe.

The transition metal complex preferably is of the general formula (AI):

[MₐLₖXₙ]Yₘ

in which:
M represents a metal selected from Mn(II)-(III)-(IV)-(V), Cu(I)-(II)-(III), Fe (II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI), preferably from Fe(II)-(III)-(IV)-(V);
L represents the ligand, preferably N,N-bis(pyridin-2-yl-methyl)-1,1-bis(pyridin-2-yl)-1-aminoethane, or its protonated or deprotonated analogue;
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;
k represents an integer from 1 to 10;
n represents zero or an integer from 1 to 10;
m represents zero or an integer from 1 to 20.

Preferably, M represents a metal selected from Fe (II)-(III)-(IV)-(V) and Co(I)-(II)-(III). More preferably, M represents a metal selected from Fe (II)-(III)-(IV)-(V).

Preferably, the complex is an iron complex comprising the ligand N,N-bis(pyridin-2-yl-methyl)-1,1-bis(pyridin-2-yl)-1-aminoethane. Suitable classes of ligands are described in the literature mentioned hereabove, in the section bleach catalyst.

Alternatively the bleach catalyst may be a redox enzyme, preferably a oxidoreductase. Examples of the oxidoreductases are oxidases such as glucose oxidase, methanol oxidase, bilirubin oxidase, catechol oxidase, laccase, peroxidases such as ligninase and those described in WO-A-97/31090, monooxygenase, dioxygenase such as lipoxygenase and other oxygenases as described in WO-A-99/02632, WO-A-99/02638, WO-A-99/02639 and the cytochrome based enzymatic bleaching systems described in WO-A-99/02641.

The activity of oxidoreductases may be enhanced by adding certain organic compounds, called enhancers. Examples of enhancers are 2,2'-azo-bis-(3-ethylbenzo-thiazoline-6-sulphonate (ABTS) and Phenothiazine-10-propionate (PTP).
More enhancers are described in WO-A-94/12619, WO-A-94/12620, WO-A-94/12621, WO-A-97/11217, WO-A-99/23887. Enhancers are generally added at a level of 0.01% to 5% by weight of the composition.

The enhancer 2,2'-azo-bis-(3-ethylbenzo-thiazoline-6-sulphonate (ABTS) changes color (becomes green) on reaction with hydroperoxides. This enhancer may therefore also function as colorant according to the invention and no additional colorant needs to be present. Also other (functional) substances in the indicator composition may function as colorant in a comparable way. Such a compound that changes its color is disclosed in D.A. Becker et al, JACS, 118, 905 (1980) and D.A.Becker, US 6,198,825 B1.

The indicator composition may comprise other functional substances, such as binders, fillers, binders, etc.

Examples of binders are animal glue, starches and gums, and synthetics, which fuse and hold the indicator composition together. Examples of fillers, mostly inert materials, are ground glass, diatomaceous earth, silica etc. The fillers provide bulk and a create a contact area for the indicator composition in contact with the oil to be tested.

The amount of the indicator composition in the device should be such, that a change of color of the indicator composition may be detected. The amount is preferably such that it is possible to see a change of color of the composition with the naked human eye.

The indicator composition may be present in the device in any suitable form, i.e. a form that allows a change in color to be detected. For instance the indicator composition may be used in the device in the form of a powder, paste, dissolved or suspended in a liquid. Preferably the indicator composition is deposited on a support. Fixed on a support, the indicator composition does not easily contaminate the frying oil which is tested, such that the device may be used in contact with frying oil that is subsequently used. More preferably, the support is a stick. A stick may have a coating with the indicator composition in one part of the stick, whereas another part serves as a handle. The main benefit of the use of a stick is that it is simple, fast, accurate and does not require special training to be used. Preferably the material of the stick is a heat resistant material. This allows the stick to be used in heated frying oil. Preferably the material has a low heat conductivity, such that a stick can be handled by a human at the same time that the stick is in contact with heated frying oil. Examples of a suitable materials for the stick is wood, paper, polymer or metal. Preferred materials are wood and/or paper.

The indicator composition may be applied onto the support by any suitable known method, such as impregnation, for instance on paper, dipping etc. Advantageously, part of the surface of the indicator composition on the support may be covered by a transparent or translucent coating, such that when the device is dipped into oil to be tested, this part retains its original color, while the remaining uncoated part of the indicator composition, in contact with the oil will change color. This makes the change in color more apparent, because the original color can be compared to the changed color in one glance. Advantageously, the support or the material on the support, such as fillers or binders, may be chemically or electostatically bound to the indicator composition or parts of the indicator composition. For instance, the bleaching catalyst may be chemically modified to react with a polymer support. Another way of fixing the indicator composition to the support is to use an inert negatively charged support or coating material, such as zeolite or silica, to which a positively charged colorant is polarly bound.

The invention further relates to a method for analysing oil quality, wherein the amount of hydroperoxides in the oil is measured and the amount of hydroperoxides used to express the oil quality. Though not wishing to be bound by theory, it is assumed that the invention works according to the following principle: In fresh frying oil, the amount of hydroperoxides is relatively low and the oil contains a relatively high amount of natural (such as phytosterols or tocopherol) and optionally added antioxidants. During use of the oil in frying, the oil will gradually decompose and oxidise and peroxides will be formed. The antioxidants in the oil will react with the peroxides, but at least as long as antioxidants are present in the oil, these will be reduced by the antioxidants. As a result, the level of free hydroperoxides in the oil remains low. Only when the oil is oxidised and all the antioxidants have reacted, the level of free hydroperoxides will increase. At this time, the device according to the invention will detect an amount of hydroperoxides, since the hydroperoxides will oxidise the colorant in a reaction that is catalysed by the bleaching catalyst.

We have found that the change of color of the indicator composition gives a good measure for detection the quality of the frying oil. We have found that when the device according to the invention is brought into contact with used frying oil, the time before change of color occurs (lag time), is an accurate measure for the amount of antioxidants in the oil. The longer the lag time, the higher the amount of antioxidants. The invention therefore also relates to a method for analysing oil quality, wherein the amount of antioxidants the oil is measured using a colorant, wherein the time prior to the start of change of color of the colorant is taken as a measure for the amount of antioxidants in the oil.

The device according to the invention may be used in the edible oil manufacturing industry, food service industry, and by the home user concerned about frying oil quality. The advantages of the invention in these uses are illustrated hereunder:
*Edible oil manufacturing industry:* the invention will provide an easy and accurate measurement of the hydroperoxide levels in edible oil during manufacturing. Formation of hydroperoxides in oil is associated with off-flavour formation and decreased healthiness. This measurement could decrease the need of conventionally used methods, such as organoleptic measurement or the oxygen measurement.
*Food Service Industry:* The invention provides a rapid, easy and accurate measurement of deep fry oil by measuring the levels of antioxidants and hydroperoxides. The prior art 3M stick needs to be stored cool, which makes the supply chain more difficult to handle. Another disadvantage is that the known method and device are difficult to use in practice. The temperature of the 3 M device needs to be carefully controlled. Further a drop of the frying oil has to be deposited on the test strip placed. Both steps may be cumbersome in a home kitchen or in a commercial kitchen, where cooking may take place at the same time.

*Home user of deep fried oils:* A device, which indicates the healthiness of the edible oil used and which indicates that the consumer should replace the oil, suits the need of home users of deep frying oil.
The invention is illustrated by the following example.

### Example

### Example 1

### Preparation of the bleaching catalyst

[Fe(L)Cl]Cl (L= N,N-bis(pyridin-2-yl-methyl)-1,1-bis(pyridin-2-yl)-1-aminoethane). The ligand L was prepared according to the procedure found in EP 0 909 809 A, example 1. The corresponding complex was synthesised as disclosed in WO 0116271, example 1(ii).

An indicator composition was prepared by dissolving in heptane, at room temperature, the following ingredients:
10 µM of the above prepared bleaching catalyst and 5µM trans-beta-carotene, ex Sigma (colorant).

1 mg of the indicator composition was added to 200 mg frying oil. The change of color of the mixture in time was followed using was UV-visible light spectrophotometry (using a Hewlett Packard diode array spectrophotometer).

Lipid hydroperoxide (HPO) amounts in the frying oil were determined according to standard iodine titrations. The HPO levels are expressed in mmol hydroperoxide (-OOH) per kg oil. The results are shown in table 1.

**Table 1:**

| Results of frying test | | | | |
|---|---|---|---|---|
| Frying oil | Times/Days of use | Lag-time (minutes) | Full carotene bleach (minutes) | HPO (mmol OOH/kg) |
| Sunflower oil | unused | > 60 | - | 6.7 |
| | 2 times* | 20 | 50 | 14.5 |
| | 4 times* | 4 | 13 | 28.4 |

| | | | | |
|---|---|---|---|---|
| * Used for 10 minutes at 170 °C and subsequently at 190°C. | | | | |

## Claims

1. Device suitable for analysing edible oil quality, **characterized in that** it comprises an indicator composition comprising a colorant and a bleach catalyst.

2. Device acording to claim 1, wherein the bleach catalyst is chosen from the group consisting of transition metal catalysts and redox enzymes.

3. Device according to claim 1 or 2, wherein the indicator composition is deposited on a support.

4. Device accoriding to claim 3, wherein the support is a stick.

5. Device according to claim 2, wherein the bleach catalyst is a transition metal catayst chosen from the group consisting of [MₐLₖXₙ]Yₘ in which:
M represents a metal selected from Mn(II)-(III)-(IV)-(V), Cu(I)-(II)-(III), Fe (II)-(III)-(IV)-(V), Co(I)-(II)-(III), Ti(II)-(III)-(IV), V(II)-(III)-(IV)-(V), Mo(II)-(III)-(IV)-(V)-(VI) and W(IV)-(V)-(VI), preferably from Fe (II)-(III)-(IV)-(V);
L represents the ligand, preferably N,N-bis(pyridin-2-yl-methyl)-1,1-bis(pyridin-2-yl)-1-aminoethane, or its protonated or deprotonated analogue;
X represents a coordinating species selected from any mono, bi or tri charged anions and any neutral molecules able to coordinate the metal in a mono, bi or tridentate manner;
Y represents any non-coordinated counter ion;
a represents an integer from 1 to 10;
k represents an integer from 1 to 10;
n represents zero or an integer from 1 to 10.

6. Device according to claim 5, wherein M represents a metal selected from Fe (II)-(III)-(IV)-(V) and Co(I)-(II)-(III).

7. Device according to claim 6, wherein M represents a metal selected from Fe (II)-(III)-(IV)-(V).

8. Device according to any of claims 1-7, wherein the colorant is a food colorant.

9. Device according to claim 8, wherein the food colorant is chosen from the group consisting of curcumin, riboflavin, Tartrazin, Chinolin, Cochenil, and beta-carotene.

10. Method for analysing oil quality, wherein the amount of hydroperoxides in the oil is measured and the amount of hydroperoxides is used to express the oil quality.

11. Method according to claim 10, wherein the amount of hydroperoxides in the oil is measured using a colorant, wherein the time needed to change the color of the colorant is taken as a measure for the amount of hydroperoxides in the oil.

12. Method for analysing oil quality, wherein the amount of antioxidants in the oil is measured and the amount of antioxidants is used to express the oil quality.

13. Method according to claim 12, wherein the time prior to the start of change of color of the colorant is taken as a measure for the amount of antioxidants in the oil.
